# EUROPEAN PATENT APPLICATION

(11) **EP 4 512 528 A1**
(43) Date of publication of application: **26.02.2025**
(21) Application number: 23219003.3
(22) Date of filing: 21.12.2023
(51) Int. Cl.: B03C 3/16, B03C 3/32, B03C 3/36, B03C 3/41, B03C 3/53, B03C 3/86

(54) **ELECTRICAL DISCHARGE TYPE AIR CLEANER**

(30) Priority: 22.08.2023 KR 20230109698
(71) Applicant: Gonggong Co., Ltd., Seoul 08503 (KR)
(72) Inventor: Lee, Sun Un, 06964 Seoul (KR)
(74) Representative: Jacobi, Markus Alexander

(57) **Abstract**

An electrical discharge type air cleaner is provided. The air cleaner includes a dust collecting tank accommodating liquid, a housing accommodating the dust collecting tank, an air supplying portion supplying air to the dust collecting tank, an air discharging portion discharging the air from the dust collecting tank and having an air discharging tube facing a surface of the liquid, a plurality of discharge electrodes disposed at the air discharging tube, a charging portion in the dust collecting tank, and a rotary vane forming a vortex in the liquid. The charging portion charges the liquid with a polarity opposite to a polarity of the discharge electrodes such that an electric field is formed between the discharge electrodes and the liquid and pollutants in the air are collected into the liquid. A distance between the surface of the liquid and the discharge electrode is maintained within a predetermined distance range.

## Description

### TECHNICAL FIELD

The present disclosure relates to an air cleaner that collects pollutants contained in air by means of liquid.

### BACKGROUND

Various types of air cleaners, which collect pollutants such as fine dust generated indoors or introduced from the outside to purify indoor air and discharge the purified air indoors, are being used. Such air cleaners may be broadly classified into dry-type air cleaners and wet-type air cleaners.

The dry-type air cleaner purifies air by means of a filter filtering pollutants or a dust collector electrically collecting pollutants. The dry-type air cleaner requires periodic replacement or cleaning of the filter or the dust collector. The replacement of the filter or the dust collector incurs additional costs, and the cleaning of the filter or the dust collector is inconvenient for a user.

The wet-type air cleaner purifies air by depositing or dissolving pollutants by bringing the air into contact with a wet depurative such as water. In the wet-type air cleaner, the small contact area between pollutant-containing air and the wet depurative reduces the air purifying performance of the wet-type air cleaner. Further, due to the wet depurative being maintained in a static state, a container accommodating the wet depurative is easily contaminated.

### SUMMARY

Embodiments of the present disclosure provide an air cleaner that solves the aforementioned problems of the prior art technique. One embodiment of the present disclosure provides an air cleaner that exhibits high air purifying performance with low power consumption in a wet environment. One embodiment of the present disclosure provides an air cleaner, whish is used in a wet environment, does not cause inconvenience such as filter replacement, and realizes automated operation.

Disclosed embodiments relate to an air cleaner which removes pollutants in air by means of electrical discharge. The air cleaner according to one embodiment includes a dust collecting tank, a housing, an air supplying portion, an air discharging portion, a plurality of discharge electrodes, a charging portion, and a rotary vane. The dust collecting tank is configured to accommodate a liquid. The housing is configured to accommodate the dust collecting tank. The air supplying portion is configured to supply air from an outside into the dust collecting tank to collect pollutants contained in the air. The air discharging portion has an air discharging tube disposed so as to face a surface of the liquid, and is configured to discharge the air supplied to the dust collecting tank to the outside. The plurality of discharge electrodes are disposed along a circumference of the air discharging tube. The charging portion is disposed in the dust collecting tank, and charges the liquid with a polarity opposite to a polarity of the plurality of discharge electrodes such that an electric field is formed between the plurality of discharge electrodes and the liquid and the pollutants are collected into the liquid. The rotary vane is installed on a bottom of the dust collecting tank, and rotates the liquid such that a vortex is formed in the liquid. The air cleaner is configured so that a spaced apart distance between the surface of the liquid and the plurality of discharge electrodes is within a predetermined distance range.

In one embodiment, the plurality of discharge electrodes are configured to be charged with a positive (+) polarity, and the charging portion is configured to charge the liquid with a negative (-) polarity.

In one embodiment, each of the plurality of discharge electrodes protrudes from the air discharging tube in a radially outward direction of a central axis of the air discharging tube.

In one embodiment, the air cleaner includes a grounding portion installed in the housing and configured to ground the dust collecting tank. The dust collecting tank may include a non-conductive material in an inner peripheral surface or an outer peripheral surface of the dust collecting tank.

In one embodiment, the air cleaner includes a rotary motor installed in the housing and configured to rotate the rotary vane. The rotary motor is configured to change a revolution number of the rotary motor so that the spaced apart distance is within the predetermined distance range.

In one embodiment, the air cleaner includes a controller configured to control the rotary motor. The rotary motor is configured to detect a revolution number of the rotary motor and to transmit the detected revolution number to the controller. The controller is configured to: compare a reference revolution number of the rotary motor, at which the spaced apart distance is allowed to be within the predetermined distance range, with the detected revolution number inputted from the rotary motor; and to increase a revolution number of the rotary motor to heighten a level of the liquid so that the spaced apart distance is within the predetermined distance range.

In one embodiment, the air cleaner includes a liquid level detecting sensor configured to detect a level of the liquid, and a controller configured to adjust the spaced apart distance based on a detection signal from the liquid level detecting sensor so that the spaced apart distance is within the predetermined distance range.

In one embodiment, the controller is configured to change a revolution number of the rotary motor so that the spaced apart distance is within the predetermined distance range.

In one embodiment, the air cleaner includes a replenishing tank configured to accommodate a replenishing liquid for replenishing the liquid in the dust collecting tank, and a pump configured to supply the replenishing liquid of the replenishing tank to the dust collecting tank. The controller is configured to control the pump so that the spaced apart distance is within the predetermined distance range.

In one embodiment, the air cleaner includes a replenishing tank configured to accommodate a replenishing liquid having a concentration different from a concentration of the liquid, a connection portion interconnecting the dust collecting tank and the replenishing tank, a semipermeable membrane installed in the connection portion, and a valve installed in the connection portion and configured to open and close the connection portion. The controller is configured to control the valve so that the spaced apart distance is within the predetermined distance range.

In one embodiment, the controller is configured to change positions of the plurality of discharge electrodes. The plurality of discharge electrodes are configured to be vertically movable so that the spaced apart distance is within the predetermined distance range.

### BRIEF DESCRIPTION OF DRAWINGS

The accompanying drawings, which are incorporated in and constitute a part of the specification, illustrate embodiments of the present disclosure, and together with the general description given above and the detailed description of the embodiments given below, serve to explain the principles of the present disclosure.
FIG. 1 is a perspective view showing an electrical discharge type air cleaner according to one embodiment.
FIG. 2 is a cross-sectional view taken along line 2-2 of FIG. 1.
FIG. 3 is a perspective view showing a plurality of discharge electrodes according to one embodiment.
FIG. 4 is a cross-sectional view similar to FIG. 2, and shows an example of a surface shape of a liquid rotated by a rotary vane.
FIG. 5 is an enlarged cross-sectional view showing a rotary vane and a rotary motor shown in FIG. 2.
FIG. 6 is a block diagram schematically showing a configuration of an operating part of an electrical discharge type air cleaner according to one embodiment including a controller.
FIG. 7 is a block diagram schematically showing a configuration of an operating part of an electrical discharge type air cleaner according to one embodiment including a controller and a liquid level detecting sensor.
FIG. 8 is a block diagram schematically showing a configuration of an operating part of an electrical discharge type air cleaner according to one embodiment in which a liquid in a dust collecting tank is replenished by a pump.
FIG. 9 is a block diagram schematically showing a configuration of an operating part of an electrical discharge type air cleaner according to one embodiment in which a liquid in a dust collecting tank is replenished by osmotic pressure.
FIG. 10 is a block diagram schematically showing a configuration of an operating part of an electrical discharge type air cleaner according to one embodiment configured to change a position of a discharge electrode.
FIG. 11 is a cross-sectional view showing a discharge electrode moving portion and an air discharging tube according to one embodiment.
FIG. 12 is a cross-sectional view of an electrical discharge type air cleaner according to one embodiment, showing an example of an arrangement of discharge electrodes.
FIG. 13 is a cross-sectional view of an electrical discharge type air cleaner according to one embodiment, showing another example of an arrangement of discharge electrodes.
FIG. 14 is a cross-sectional view of an electrical discharge type air cleaner according to one embodiment, showing yet another example of an arrangement of discharge electrodes.

### DETAILED DESCRIPTION

Embodiments of the present disclosure are illustrated for the purpose of explaining the technical idea of the present disclosure. The scope of the rights according to the present disclosure is not limited to the embodiments presented below or the detailed descriptions of such embodiments.

All technical terms and scientific terms used in the present disclosure include meanings that are commonly understood by those of ordinary skill in the technical field to which the present disclosure pertains unless otherwise defined. All terms used in the present disclosure are selected for the purpose of describing the present disclosure more clearly, and are not selected to limit the scope of the rights according to the present disclosure.

Expressions such as "comprising," "including," "having," and the like used in the present disclosure are to be understood as open-ended terms having the possibility of encompassing other embodiments, unless otherwise mentioned in a phrase or sentence containing such expressions.

Singular expressions that are described in the present disclosure may encompass plural expressions unless otherwise stated, which will also be applied to the singular expressions recited in the claims.

Expressions such as "first," "second," etc. used in the present disclosure are used to distinguish a plurality of elements from each other, and are not intended to limit an order or importance of the elements.

In the present disclosure, the description that one element is "connected" or "coupled" to another element should be understood to indicate that one element may be directly connected, or coupled, to another element, or that a new different element may be interposed between the one element and the another element to be connected or coupled thereby.

Hereinafter, the embodiments are described with reference to the accompanying drawings. Like reference numerals in the accompanying drawings denote like or corresponding elements. Further, in the following description of the embodiments, redundant descriptions for the same or corresponding elements may be omitted. However, even if the descriptions of the elements are omitted, such elements are not intended to be excluded in any embodiment.

The embodiments described hereinafter and the embodiments shown in the accompanying drawings relate to an electrical discharge type air cleaner, which purifies air by ionizing pollutants contained in air by means of electrical discharge and collecting the ionized pollutants by a charged liquid. The electrical discharge type air cleaner (hereinafter, simply referred to as an "air cleaner") according to the present disclosure may be used so as to remove pollutants contained in indoor air, but the use location of the air cleaner according to the present disclosure is not limited to an indoor location.

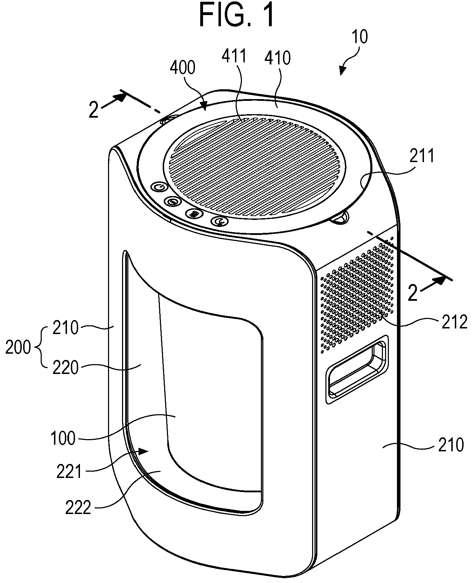

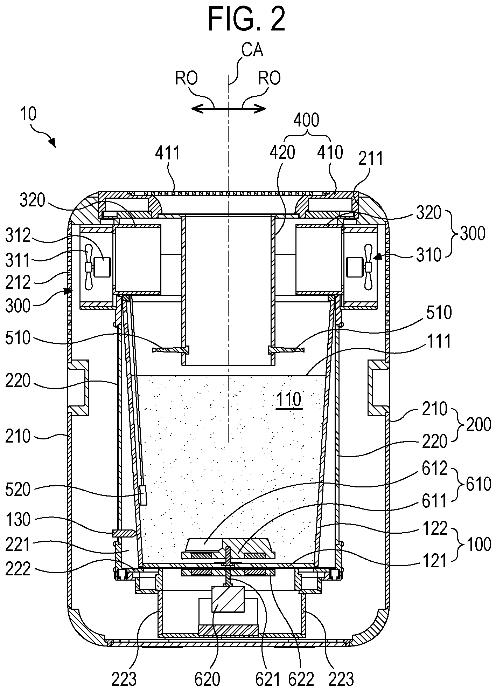

FIG. 1 is a perspective view showing an air cleaner according to one embodiment of the present disclosure, and FIG. 2 is a cross-sectional view taken along line 2-2 of FIG. 1. FIGS. 1 and 2 are referred to together in the following description.

The air cleaner 10 according to one embodiment includes a dust collecting tank 100 configured to accommodate a liquid 110, and a housing 200 accommodating the dust collecting tank 100.

The liquid 110 collects pollutants contained in air (e.g., indoor air). The pollutants contained in the air may be, for example, fine dust (PM10, PM2.5, etc.), formaldehyde, radon, hydrocarbons, nitrogen oxides, sulfur oxides, heavy metals, carbon monoxide, or the like. The liquid accommodated in the dust collecting tank 100 may include a liquid having a low vapor pressure, such as water, oil, or ether, in order to collect the pollutants contained in the air. To enhance pollutant-collecting performance or to reduce evaporation of the liquid, an additive may be added to the liquid.

The dust collecting tank 100 may be made of a transparent material, and may have a shape such as an inverted truncated cone having a diameter gradually increasing upward. The dust collecting tank 100 may be installed in the housing 200 so as to be removably fixed to the housing. By way of example, the dust collecting tank 100 may have a circular bottom wall 121 and a cylindrical side wall 122. The dust collecting tank 100 is accommodated in the housing 200 and is supported by the housing 200.

The housing 200 serves as a support accommodating components of the air cleaner 10, and holds and supports the dust collecting tank 100. By way of example, the housing 200 includes an outer housing 210 forming an exterior of the air cleaner 10, and an inner housing 220 located inside the outer housing 210 and fixed to the outer housing 210. The outer housing 210 has an opening 211 perforated at an upper end thereof, and a plurality of air suction holes 212 perforated in a side wall thereof. The outer housing 210 may be placed on an indoor floor, or on a surface of a suitable structure. The inner housing 220 defines an exposure opening 221 perforated in the inner housing in a front and rear direction of the air cleaner. The dust collecting tank 100 is positioned in the exposure opening 221 and may be fixed to a bottom wall 222 of the inner housing 220.

The air cleaner 10 includes an air supplying portion 300 and an air discharging portion 400. The air supplying portion 300 and the air discharging portion 400 are installed in the housing. The air supplying portion 300 is configured to supply air from the outside of the air cleaner 10 to the dust collecting tank 100 in order to purify the air by collecting the pollutants contained in the air by the liquid 110. The air discharging portion 400 is configured to discharge the air, which is supplied to the dust collecting tank 100 and from which the pollutants are collected by the liquid 110, from the dust collecting tank 100 to the outside of the air cleaner 10.

The air supplying portion 300 is located inside the outer housing in the vicinity of a top portion of the outer housing. The air supplying portion 300 may be fixed to the inside of the outer housing 210 between the outer housing 210 and the inner housing 220. By way of example, the air supplying portion 300 may have an air suction device 310 configured to suction the air containing pollutants from the outside of the housing 200, and an air supplying tube 320 extending from the air suction device 310. The air suction device 310 may have an air suction fan 311 disposed to face the air suction hole 212 and a fan motor 312 rotating the air suction fan 311. As the air suction fan 311 is rotated by the fan motor 312, the air in the outside of the air cleaner can be supplied to the air supplying tube 320 through the air suction device 310. An operation of the fan motor 312 may be controlled by a controller of the air cleaner. By way of example, by increasing or decreasing a revolution number of the air suction fan 311 rotated by the fan motor 312, the amount of air supplied to the dust collecting tank 100 can be adjusted and a dust collection speed of pollutants can be adjusted. The air supplying tube 320 guides the air blown by the air suction device 310 toward the dust collecting tank 100. The air supplying tube 320 is disposed to face the inside of the inner housing 220. By way of example, the air supplying tube 320 may extend from the air suction device 310 toward the dust collecting tank 100. By way of another example, the air supplying tube 320 may extend straight or obliquely from the air suction device 310 toward the air discharging portion 400, and the air blown from the air supplying tube 320 may be guided to the dust collecting tank 100 along the air discharging portion 400.

The air discharging portion 400 discharges the air, from which the pollutants are removed by being collected to the liquid 110, to the outside of the air cleaner. The air discharging portion 400 is located above the dust collecting tank 100 and may be coupled to the top portion of the outer housing 210. The air discharging portion 400 is configured to discharge the air in the dust collecting tank 100, from which the pollutants are removed, upward from the dust collecting tank 100. By way of example, the air discharging portion 400 may be configured as a modularized component, and may be removably coupled to the top portion of the outer housing 210. Accordingly, in a state where the air discharging portion 400 is removed from the housing 200, the dust collecting tank 100 located in the inner housing 220 and the liquid 110 in the dust collecting tank 100 can be exposed. In such a state, replenishment of the liquid 110 may be performed through the opening 211 of the outer housing 210.

The air discharging portion 400 has a cover portion 410 coupled to the opening 211 of the outer housing 210 and an air discharging tube 420 extending downward from the cover portion 410. The cover portion 410 has, in its top portion, a plurality of air discharging holes 411, and the air from which the pollutants are removed is discharged through the air discharging holes 411. The air discharging tube 420 extends downward from a bottom portion of the cover portion 410, and the cover portion 410 has an interior space communicating with the air discharging tube 420. The air discharging tube 420 is formed in the air discharging portion 400 so as to be disposed coaxially with a central axis CA of the dust collecting tank 100. The air discharging tube 420 is disposed in the air discharging portion 400 so as to face a surface 111 of the liquid 110 in a downward direction. Thus, the air discharging portion 400 is coupled to the housing 200 such that the air discharging tube 420 is spaced upward from the surface 111 of the liquid 110. A lower end of the air discharging tube 420 faces the surface of the liquid 110.

The air cleaner 10 according to one embodiment ionizes or charges the pollutants in the air supplied through the air supplying portion 300 with a positive (+) polarity or a negative (-) polarity by electrical discharge, and charges the liquid 110 collecting the pollutants with a polarity opposite to the polarity of the pollutants. Therefore, the pollutants in the air supplied to the dust collecting tank 100 are collected to the liquid 110 in the dust collecting tank 100. For the above-described collection of the pollutants, the air cleaner 10 includes a discharge electrode 510 configured to perform electrical discharge in the air introduced into the dust collecting tank 100, and a charging portion 520 configured to electrically charge the liquid 110.

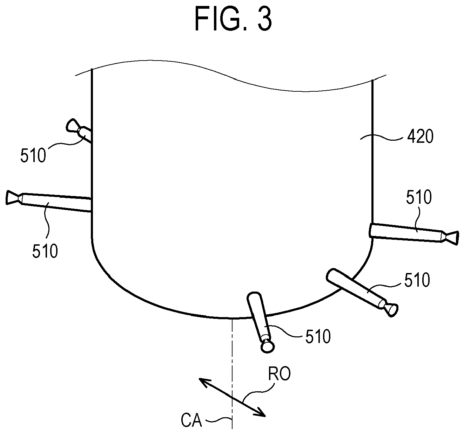

FIG. 3 is a perspective view showing a plurality of discharge electrodes disposed in the air discharging tube of the air discharging portion according to one embodiment. Reference is made to FIGS. 2 and 3 together in the following description.

A plurality of discharge electrodes 510 are disposed along a circumference of the air discharging tube 420. By way of example, four to six discharge electrodes 510 may be disposed along the circumference of the air discharging tube 420, but the number of discharge electrodes is not limited thereto. The plurality of discharge electrodes 510 are disposed at equal or predetermined intervals along the circumference of the air discharging tube 420 around the air discharging tube 420. By way of example, each discharge electrode 510 may be made of carbon fiber. The carbon fiber discharge electrode may be used at low voltage and low power consumption, and may have a low heat generation amount. In the embodiments of the air cleaner, the plurality of discharge electrodes 510 may be disposed along the circumference of the air discharging tube 420 in a state of being coupled to the air discharging tube 420, the air discharging portion 400, or the dust collecting tank 100.

In the embodiment shown in FIGS. 2 and 3, the plurality of discharge electrodes 510 are coupled to the air discharging tube 420 such that each protrudes from an outer peripheral surface of the air discharging tube 420 in a radially outward direction RO of the central axis CA of the air discharging tube. The plurality of discharge electrodes 510 are disposed at equal or predetermined intervals along the circumference of the air discharging tube 420 in the vicinity of the lower end of the air discharging tube 420. Since the plurality of discharge electrodes 510 protrude in all directions with respect to a center of the air discharging tube 420, discharge efficiency does not decrease in a high humidity environment.

Referring to FIG. 2, the charging portion 520 is disposed to be submerged in the liquid 110 in the dust collecting tank 100 and charges the liquid 110. The charging portion 520 is configured to charge the liquid 110 with a polarity opposite to the polarity with which the plurality of discharge electrodes 510 are charged. As the charging portion 520 charges the liquid 110, an electric field is formed between the plurality of discharge electrodes 510 and the liquid 110, and thus pollutants are collected in the liquid 110. The charging portion 520 may include a charging electrode submerged in the liquid 110, and an electric wire connected to the charging electrode may be attached to an inner peripheral surface of the dust collecting tank 100 and extend to the inside of the housing 200.

According to one embodiment, the plurality of discharge electrodes 510 are configured to be charged with a positive (+) polarity, and the charging portion 520 is configured to charge the liquid 110 with a negative (-) polarity. Due to the plurality of discharge electrodes 510 charged with the positive (+) polarity, the pollutants located around the discharge electrodes are electrically destroyed and ionized to positive ions. For example, a positive corona discharge may be generated at the plurality of discharge electrodes 510. The pollutants ionized to positive ions are attracted to the liquid 110 charged with the negative (-) polarity by, for example, a force generated by an electric field, electrostatic attraction, or the like, and can be collected into the liquid 110. Since the discharge electrodes 510 are charged with the positive (+) polarity, the amount of ozone formed when oxygen radicals generated at the discharge electrodes combine with oxygen in the air is small. As another example, the plurality of discharge electrodes 510 may be charged with a negative (-) polarity, the charging portion 520 may charge the liquid 110 with a positive (+) polarity, and the pollutants may be ionized to negative ions by the discharge electrodes 510.

According to some embodiments, the liquid 110 in the dust collecting tank 100 may be accommodated in the dust collecting tank 100 so as not to have fluidity. As the air cleaner 10 operates, the pollutants may be accumulated on the surface of the liquid 110, dust collection efficiency may be reduced, and the collected pollutants may electrically connect the discharge electrodes 510 and the liquid 110. The air cleaner 10 according to one embodiment rotates the liquid 110 in the dust collecting tank to allow a vortex to be formed in the liquid 110. Therefore, the surface 111 of the liquid 110 can be modified, reduction in dust collection efficiency can be prevented, and an electrical short circuit caused by pollutants can be avoided.

The air cleaner 10 according to one embodiment includes a rotary vane 610, which is installed on the bottom wall 121 of the dust collecting tank 100 and rotates the liquid 110. The rotary vane 610 is rotatably coupled to the bottom wall 121 of the dust collecting tank 100 so as to rotate about the central axis CA of the dust collecting tank 100. By way of example, the rotary vane 610 has a base portion 611 formed in a circular disk shape, and a plurality of vane portions 612 protruding upward from the base portion 611. Each vane portion 612 may be formed to extend from the vicinity of a rotation center of the base portion 611 to an edge of the base portion 611.

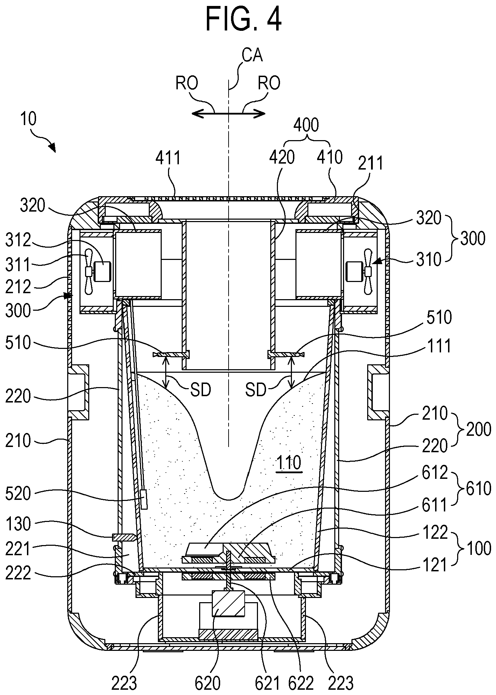

FIG. 4 is a cross-sectional view similar to FIG. 2, and shows an example of a shape of the surface of the liquid rotated by the rotary vane. Reference is made to FIGS. 2 and 4 together in the following description.

As the rotary vane 610 rotates about the central axis CA, a vortex is formed in the liquid 110. As the rotary vane 610 rotates, a height of the surface 111 of the liquid 110 can be heightened, and the vortex rotating about the central axis CA is formed in the liquid 110. In the liquid 110 where the vortex is formed, the surface 111 may take an approximately V-shape that is concave downward. The rotary vane 610 rotates the liquid 110 so that the surface 111 of the liquid 110 with the vortex formed therein is located close to, but does not make contact with tip portions of the discharge electrodes 510. Since the vortex is formed in the liquid 110 and the surface 111 of the liquid 110 changes from a flat shape to a downward concave shape, the contact area between the pollutant-containing air and the surface 111 of the liquid 110 increases. That is, as the rotary vane 610 rotates, the surface 111 can have an increased contact area, and the dust collection efficiency of pollutants can be further increased. Further, since the liquid 110 with the vortex formed therein can wash out the dust collecting tank 100, internal contamination of the dust collecting tank can be reduced, and water scale formation in the dust collecting tank due to the collected pollutants can be prevented. Further, the surface 111 of the liquid 110 can be modified, and an electrical short circuit between the discharge electrodes and the liquid due to the pollutants can be prevented.

The air cleaner 10 according to one embodiment includes a rotary motor 620 configured to rotate the rotary vane 610. In some embodiments, the air cleaner may not have the rotary motor 620 for rotating the rotary vane 610. The rotary motor 620 is installed in the housing 200 and may be directly or indirectly connected to the rotary vane 610. A rotary shaft of the rotary motor 620 and a rotary shaft of the rotary vane 610 may be directly connected to each other. In such a case, one of the rotary shaft of the rotary motor 620 and the rotary shaft of the rotary vane 610 may pass through the bottom wall 121 of the dust collecting tank 100, and a sealing member for preventing leakage of the liquid may be interposed between the bottom wall 121 of the dust collecting tank and the rotary shaft. The rotary shaft of the rotary motor 620 and the rotary shaft of the rotary vane 610 may be separated from each other, and a rotation force of the rotary motor 620 may be delivered to the rotary vane 610 in a non-contact way. In such a case, the rotary shaft passing through the bottom wall 121 of the dust collecting tank 100 and the sealing member for the same are not required, and leakage of liquid can be prevented.

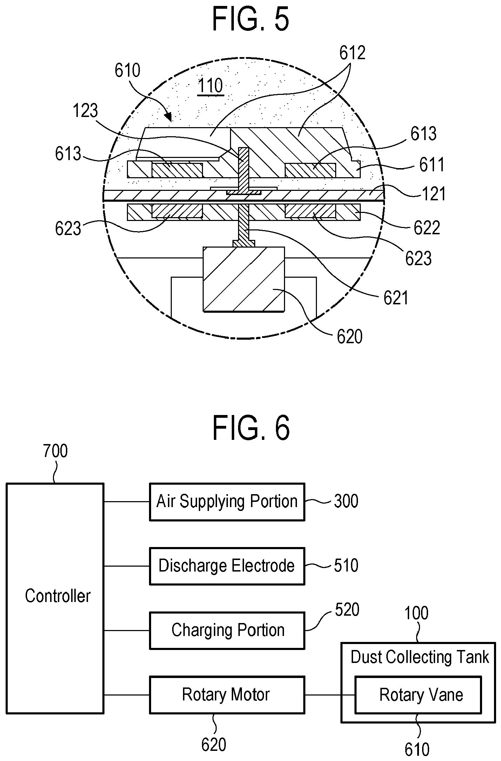

FIG. 5 is an enlarged cross-sectional view showing the rotary vane and the rotary motor shown in FIG. 2. Reference is made to FIGS. 2 and 5 together in the following description.

The inner housing 220 has a motor accommodating portion 223 that is concavely formed downward from the bottom wall 222. The rotary motor 620 is installed in the motor accommodating portion 223 of the inner housing 220. The bottom wall 121 of the dust collecting tank 100 is closed, and a support shaft 123 protrudes from the bottom wall 121. The base portion 611 of the rotary vane 610 is coupled to the support shaft 123 so as to rotate around the support shaft 123. The rotary motor 620 has a driving plate 622, which is coupled to an end portion of the rotary shaft 621 and has a circular disk shape. The driving plate 622 may have approximately the size of the base portion 611 of the rotary vane.

The driving plate 622 is configured to rotate the rotary vane 610 by magnetic force. For rotation of the rotary vane 610 caused by rotation of the driving plate 622, the driving plate 622 may have a plurality of magnets 623 arranged so as to be spaced apart from each other in a circumferential direction of the driving plate, and the rotary vane 610 may have, in the base portion 611, a plurality of metal portions 613 corresponding to the magnets 623, respectively. Alternatively, the base portion 611 of the rotary vane 610 may have the plurality of magnets 623 arranged so as to be spaced apart in the circumferential direction, and the driving plate 622 may have the plurality of metal portions 613 corresponding to the plurality of magnets, respectively. Alternatively, a plurality of pairs of magnets opposing each other in a vertical direction may be installed in the driving plate 622 and the base portion 611. Thus, the rotation force of the rotary motor 620 can be delivered to the rotary vane 610 by magnetic interaction between the driving plate 622 and the base portion 611, and the rotary vane 610 separated from the rotary motor 620 can be rotated by the rotation of the rotary motor 620.

The air cleaner 10 according to one embodiment may include a grounding portion 130 installed in the housing 200 and configured to ground the dust collecting tank 100. Referring to FIG. 2, the grounding portion 130 may be installed in the inner housing 220 such that a ground terminal thereof protrudes from the exposure opening 221 toward the dust collecting tank 100. The ground terminal of the grounding portion 130 is in contact with a surface of the dust collecting tank 100. Specifically, the grounding portion 130 is positioned such that the ground terminal is in contact with the vicinity of the bottom wall 121 of the dust collecting tank 100. As another example, the grounding portion 130 may be installed in the inner housing 220 such that the ground terminal is in contact with a lower surface of the bottom wall 121 of the dust collecting tank 100. The liquid 110 is charged by the charging portion 520 during the operation of the air cleaner 10, but the dust collecting tank 100 is grounded by the grounding portion 130, thus enhancing safety for a user.

The charging portion 520 for charging the liquid 110 and the grounding portion 130 for grounding the dust collecting tank 100 are vertically separated from each other. By way of example, the charging portion 520 may be positioned above the grounding portion 130. Further, the charging portion 520 and the grounding portion 130 may be disposed in the dust collecting tank 100 so as to be electrically separated from each other. In this regard, a portion of the dust collecting tank 100 may be made of a non-conductive material. By way of example, the dust collecting tank 100 may include a non-conductive material in its inner peripheral surface or outer peripheral surface, and the non-conductive material may be formed as a portion of the dust collecting tank or be coated on the inner peripheral surface or the outer peripheral surface of the dust collecting tank.

When the air cleaner 10 operates, the pollutants are ionized due to electrical discharge of the discharge electrodes 510, and the liquid 110 is charged by the charging portion 520. Further, the vortex is formed in the liquid 110 in the dust collecting tank by the rotary vane 610. The discharge electrodes 510 are charged with the positive (+) polarity such that the electrical discharge having the positive (+) polarity is performed, and the pollutants are ionized to positive ions. By way of example, the electrical discharge having the positive (+) polarity may last for several microseconds, and the duration time of the state where the pollutants are ionized to positive ions may be very short. Accordingly, it is important to maintain an appropriate distance between the discharge electrodes 510 and the surface 111 of the liquid during the operation of the air cleaner 10.

As the air cleaner 10 operates for a predetermined period of time, the height of the surface 111 of the liquid 110 is lowered due to a natural vaporization phenomenon occurring in the liquid 110. In such a case, the appropriate distance between the discharge electrodes 510 and the liquid surface 111 is not maintained and increases, so the ionized pollutants are not be smoothly guided to the liquid 110, and the dust collection performance may deteriorate. In this regard, the air cleaner 10 according to one embodiment is configured to maintain a spaced apart distance between the surface 111 of the liquid and the discharge electrodes 510, that is, so that the spaced apart distance is within a predetermined distance range. The spaced apart distance may mean the shortest distance between the discharge electrode 510 and the surface 111 of the liquid 110 with the vortex formed therein, as shown by symbol SD in FIG. 4. By way of example, the predetermined distance range may be 3 cm to 4 cm, but may vary depending on factors such as an electric power applied to the discharge electrode, a volume of the liquid, a size of the dust collecting tank, etc.

According to one embodiment of the present disclosure, to maintain the spaced apart distance within the predetermined distance range, the evaporation amount of the liquid in the dust collecting tank can be suppressed and a change in the liquid level can be minimized. By way of example, the liquid collecting the pollutants may contain an additive such as salt, and can have a low amount of evaporation thereby. By way of another example, the liquid collecting the pollutants may be cooled, and a cooler for cooling the liquid may be installed in the housing so as to make contact with the dust collecting tank.

According to one embodiment of the present disclosure, the operation of maintaining the spaced apart distance within the predetermined distance range may be realized by following methods: a method of adjusting the spaced apart distance by increasing a rotation speed of the liquid 110 as a level of the liquid 110 (the height of the surface 111 with respect to to the bottom wall 121 of the dust collecting tank) is lowered; a method of adjusting the spaced apart distance by supplying a replenishing liquid to the dust collecting tank 100 so that the level of the liquid 110 is maintained at a constant height; a method of adjusting the spaced apart distance and the discharge electrodes 510 by moving the discharge electrodes 510; and a method of adjusting the spaced apart distance by moving the air discharging tube 420 where the discharge electrodes 510 are disposed. Further, a liquid level detecting sensor configured to detect the level of the liquid 110 may be employed in the air cleaner 10, and the spaced apart distance may be adjusted based on a signal from the sensor. Further, the above-described methods may be automatically performed by control of a controller installed inside the air cleaner 10.

According to one embodiment, the rotary motor 620 for rotating the rotary vane 610 is configured to change its revolution number. Specifically, referring to FIG. 4, the rotary motor 620 is configured to change its revolution number so that the spaced apart distance SD is within the predetermined distance range. As the level of the liquid 110 is lowered due to the operation of the air cleaner 10, the rotary motor 620 may increase its revolution number. The vortex having a higher speed can be formed in the liquid 110, and the level of the liquid 110 can be heightened by the increase in the speed of the vortex. Therefore, the spaced apart distance (SD) can be maintained within the predetermined distance range.

The air cleaner 10 may include a controller that controls components pertaining to an operating part of the air cleaner. The controller may include a processing processor and various electrical and electronic components, and may be installed inside the housing 200 or outside the housing 200. The controller may be connected to a power source for driving the components of the operating part of the air cleaner. The controller may be configured to control the operation of the air supplying portion, the operation of the discharge electrodes, the operation of the charging portion, the operation of the rotary motor, or the like. FIG. 6 is a block diagram schematically showing a configuration of an operating part of the air cleaner including a controller according to one embodiment. Reference is made to FIGS. 4 and 6 together in the following description.

A controller 700 is configured to control the rotary motor 620. The rotary motor 620 is configured to detect a revolution number of the rotary shaft 621 of the rotary motor and to transmit the detected revolution number to the controller 700. In this regard, the rotary motor 620 may have a sensor or an encoder for detecting the revolution number of the rotary shaft 621. The controller 700 may store a reference revolution number of the rotary motor 620 at which the spaced apart distance SD is maintained within the predetermined distance range. When the level of the liquid is lowered due to the operation of the air cleaner, the controller 700 may compare the reference revolution number and the detected revolution number, and may control the rotary motor 620 such that higher electrical power is applied to the rotary motor 620. For example, based on the result of comparison, the controller 700 may increase the revolution number of the rotary motor 620 to heighten the level of the liquid 110 so that the spaced apart distance SD is maintained within the predetermined distance range.

By way of example, due to the evaporation of the liquid, the liquid, whose volume is decreased, applies a reduced load to the rotary vane. Since the rotary motor 620 is supplied with electrical power so as rotate the rotary shaft 621 at the reference revolution number, the detected revolution number of the rotary shaft 621 of the rotary motor 620 may be greater than the reference revolution number in a state where the volume of the liquid is decreased and the load is reduced accordingly. Therefore, when the controller 700 determines that the detected revolution number is greater than the reference revolution number, in order to rotate the rotary vane 610 at a higher speed to heighten the level of the liquid, the controller 700 may control the rotary motor 620 to rotate at a revolution number higher than the detected revolution number by increasing the electrical power supplied to the rotary motor 620 to be higher than the electrical power applied according to the reference revolution number.

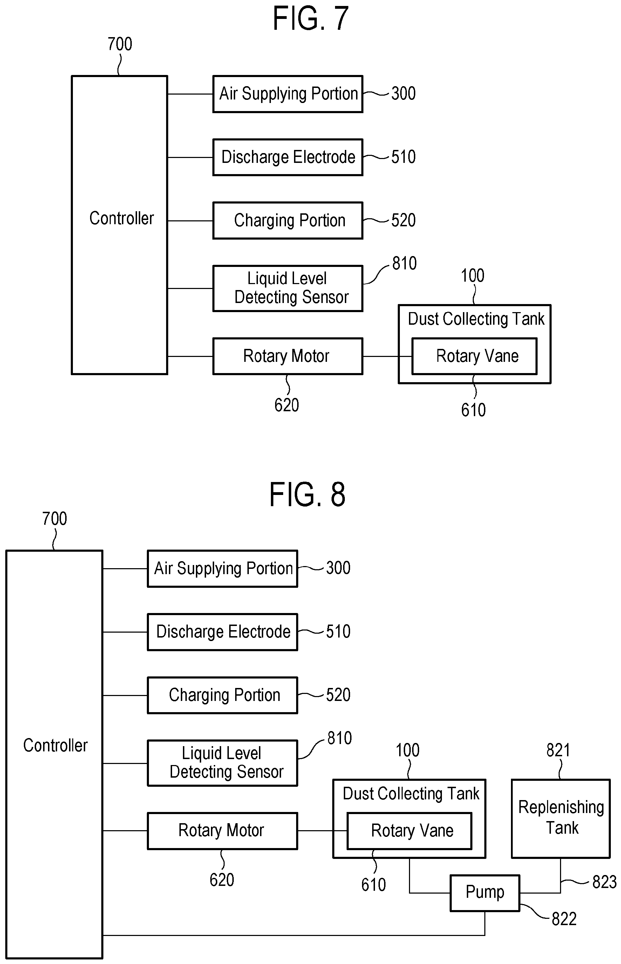

The air cleaner may include a liquid level detecting sensor configured to detect the level of the liquid, and the controller of the air cleaner may maintain the spaced apart distance based on a detection signal from the liquid level detecting sensor. FIG. 7 is a block diagram schematically showing a configuration of an operating part of an air cleaner according to one embodiment including a controller and a liquid level detecting sensor. Reference is made to FIGS. 4 and 7 together in the following description.

The liquid level detecting sensor 810 is configured to detect the level of the liquid 110 and transmit a detection signal to the controller 700. The controller 700 is configured to adjust the spaced apart distance SD so that the spaced apart distance SD is maintained within the predetermined distance range, based on the detection signal from the liquid level detecting sensor 810. The liquid level detecting sensor 810 may be installed in the dust collecting tank 100 or in the housing 200 above the dust collecting tank 100. By way of example, the liquid level detecting sensor 810 may be a sensor measuring the amount of the liquid 110 by means of an electrical resistance change, a sensor measuring the spaced apart distance SD by means of microwave pulses, ultrasonic waves, infrared rays, or the like, or a sensor detecting the level of the liquid level by means of a float, but is not limited thereto.

By way of example, the controller 700 may adjust the spaced apart distance (SD) by changing the revolution number of the rotary motor 620. The controller 700 is configured to store a reference liquid level of the liquid 110, at which the spaced apart distance SD is within the predetermined distance range, to compare a detection signal from the liquid level detecting sensor 810 with the reference liquid level, and to control the rotary motor 620 to increase the revolution number of the rotary motor 620 when the liquid level according to the detection signal is lower than the reference liquid level. Further, when the liquid level according to the detection signal is higher than the reference liquid level, the controller 700 may be configured to reduce the revolution number of the rotary motor 620 so that the spaced apart distance SD is within the predetermined distance range.

The air cleaner may maintain the spaced apart distance by replenishing the liquid in the dust collecting tank by means of a pump based on the detection signal of the liquid level detecting sensor. FIG. 8 is a block diagram schematically showing a configuration of an operating part of an air cleaner according to one embodiment in which the liquid in a dust collecting tank is replenished by a pump. Reference is made to FIGS. 4 and 8 together in the following description.

The air cleaner 10 may include a replenishing tank 821 that accommodates a replenishing liquid for replenishing the liquid 110 in the dust collecting tank 100, and a pump 822 configured to supply the replenishing liquid of the replenishing tank 821 to the dust collecting tank 100 and to be controlled by the controller 700. The liquid 110 in the dust collecting tank 100 and the replenishing liquid in the replenishing tank 821 may be a same liquid. The controller 700 may be configured to control the pump 822 so that the spaced apart distance SD is maintained within the predetermined distance range. Specifically, the controller 700 may be configured to compare a detection signal from the liquid level detecting sensor 810 with the reference liquid level, and control the pump 822 to supply the replenishing liquid in the replenishing tank 821 to the dust collecting tank 100 when the liquid level according to the detection signal is lower than the reference liquid level. Further, when the liquid level according to the detection signal approaches the reference liquid level after an operation of the pump 822, the controller 700 may stop the operation of the pump 822.

By way of example, the replenishing tank 821 may be spaced apart from the dust collecting tank 100, and may be disposed in the housing 200 or in a separate housing different from the housing 200. The replenishing tank 821 and the dust collecting tank 100 may be connected to each other via a connection pipe 823 where the pump 822 is disposed. By way of another example, the dust collecting tank 100 may be configured such that a first portion configured to accommodate the liquid 110 collecting the pollutants and to form the vortex in the liquid 110, and a second portion configured to accommodate the replenishing liquid and to surround or be coupled to the first portion are integrally formed. In such an example, a single dust collecting tank may be formed in a dual structure, which has a portion accommodating the liquid 110 for collecting the pollutants and another portion accommodating the replenishing liquid.

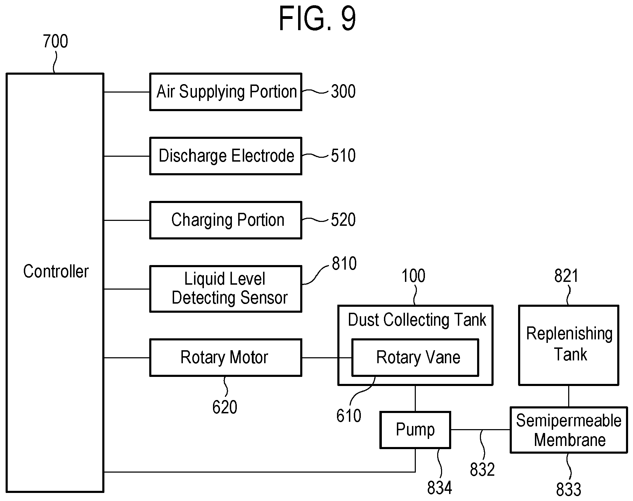

The air cleaner may maintain the spaced apart distance by replenishing the liquid in the dust collecting tank by means of osmotic pressure based on the detection signal of the liquid level detecting sensor. FIG. 9 is a block diagram schematically showing a configuration of an operating part of an air cleaner according to one embodiment, which is configured to replenish the liquid in a dust collecting tank by osmotic pressure. Reference is made to FIGS. 4 and 9 together in the following description.

The air cleaner 10 may include a replenishing tank 831 that accommodates a replenishing liquid having a concentration different from the concentration of the liquid 110 in the dust collecting tank 100, a connection portion 832 that interconnects the dust collecting tank 100 and the replenishing tank 831, a semipermeable membrane 833 installed in the connection portion 832, and a valve 834 installed in the connection portion 832, configured to open and close the connection portion 832, and controlled by the controller 700. The liquid 110 in the dust collecting tank 100 and the replenishing liquid in the replenishing tank 831 may be a same liquid having different concentrations.

The controller 700 may be configured to control the valve 834 so that the spaced apart distance SD is maintained within the predetermined distance range. Specifically, the controller 700 may be configured to compare the detection signal from the liquid level detecting sensor 810 with the reference liquid level, and to control the valve 834 to open the connection portion 832 when the liquid level according to the detection signal is lower than the reference liquid level. By way of example, when the concentration of the replenishing liquid is higher than the concentration of the liquid 110 in the dust collecting tank 100, the liquid 110 in the dust collecting tank 100 can be replenished by the action of osmotic pressure together with the opening of the valve 834. By way of another example, when the concentration of the replenishing liquid is lower than the concentration of the liquid 110 in the dust collecting tank 100, the liquid 110 in the dust collecting tank 100 can be replenished by the action of reverse osmotic pressure together with the opening of the valve 834. In such an example, a pressure may be applied to the replenishing liquid in the replenishing tank 831 for the action of reverse osmotic pressure. When the liquid level according to the detection signal approaches the reference liquid level after the operation of the valve 834, the controller 700 may control the valve 834 to close the connection portion 832.

By way of example, the replenishing tank 831 may be spaced apart from the dust collecting tank 100, and may be disposed in the housing 200 or in a separate housing different from the housing 200. The replenishing tank 831 and the dust collecting tank 100 may be connected to each other via the connection portion 832 where the semipermeable membrane 833 and the valve 834 are disposed. By way of another example, the dust collecting tank 100 may be configured such that a first portion configured to accommodate the liquid 110 collecting the pollutants and to form the vortex in the liquid 110, and a second portion configured to accommodate the replenishing liquid having a concentration different from that of the liquid 110 and to surround or be coupled to the first portion are integrally formed. In such an example, a single dust collecting tank may be formed in a dual structure, which has a portion accommodating the liquid 110 for collecting the pollutants and another portion accommodating the replenishing liquid.

In another embodiment, the dust collecting tank 100 and the replenishing tank 831 may be connected via the connection portion 832 including only the valve 834, and the replenishing liquid may be accommodated in the replenishing tank 831 so as to have a level higher than the level of the liquid in the dust collecting tank 100. In such an example, in order to adjust the spaced apart distance, the controller 700 may control the valve 834 to open and close the connection portion 832, and the liquid 110 in the dust collecting tank 100 may be replenished by the replenishing liquid in the replenishing tank 831 due to a liquid level difference.

According to a further embodiment, the dust collecting tank accommodating the liquid collecting the pollutants may be configured to change its inner diameter. When the inner diameter of the dust collecting tank is reduced, the level of the liquid with the vortex formed therein may be heightened. For example, an upper portion of the dust collecting tank may be made of a flexible material such as silicone. The upper portion of such a dust collecting tank may be reduced by a user or the controller, and the inner diameter of the dust collecting tank may be changed thereby.

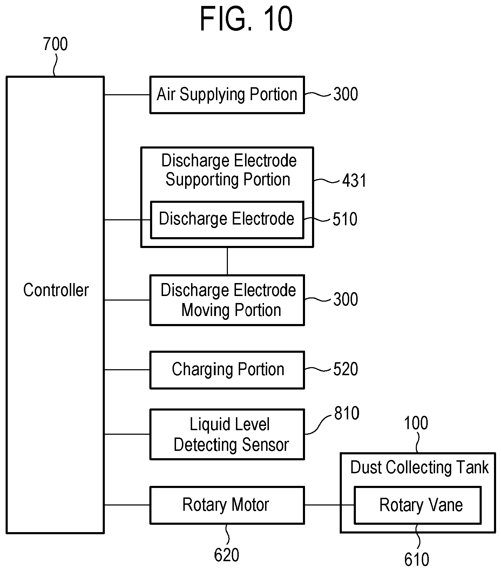

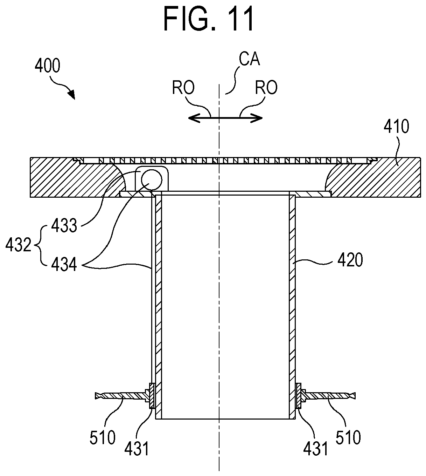

The air cleaner may maintain the spaced apart distance by changing the position of the discharge electrode based on the detection signal of the liquid level detecting sensor. FIG. 10 is a block diagram schematically showing a configuration of an operating part of an air cleaner according to one embodiment, which is configured to change a position of a discharge electrode. FIG. 11 is a cross-sectional view showing a discharge electrode moving portion and an air discharging tube according to one embodiment. Reference is made to FIGS. 4, 10, and 11 together in the following description.

The controller 700 may be configured to change the positions of the plurality of discharge electrodes 510. By the controller 700, the plurality of discharge electrodes 510 may be configured to change their positions with respect to a surface of the liquid 110. To this end, the plurality of discharge electrodes 510 may be configured to be vertically movable with respect to the air discharging tube 420 so that the spaced apart distance SD is maintained within the predetermined distance range.

Regarding the vertical movement of the discharge electrodes, the air cleaner 10 may include a discharge electrode supporting portion 431 coupled to the air discharging tube 420 so as to be vertically movable along the air discharging tube, and a discharge electrode moving portion 432 configured to raise and lower the discharge electrode supporting portion 431.

The discharge electrodes 510 are coupled to the discharge electrode supporting portion 431 so as to protrude from the discharge electrode supporting portion 431 in a radially outward direction

RO with respect to the central axis CA of the air discharging tube 420. The discharge electrode supporting portion 431 has a ring shape and supports the discharge electrodes 510. The discharge electrode supporting portion 431 may have a predetermined width in the vertical direction such that the discharge electrode supporting portion does not tilt with respect to the air discharging tube 420. The discharge electrode supporting portion 431 may be coupled to the outer peripheral surface of the air discharging tube 420 so as to be vertically movable or slidable. As the discharge electrode supporting portion 431 is moved vertically, the spaced apart distance SD can be adjusted to be within the predetermined distance range. In order to minimize friction between the discharge electrode supporting portion 431 and the air discharging tube 420, the discharge electrode supporting portion 431 may have a roller in its inner peripheral surface, the discharge electrode supporting portion 431 may be made of a material exhibiting low friction, or a lubricant such as grease may be applied between the discharge electrode supporting portion 431 and the air discharging tube 420.

The discharge electrode moving portion 432 is controlled by the controller 700. When the liquid level according to the detection signal of the liquid level detecting sensor 810 is lower than the reference liquid level, the controller 700 may control the discharge electrode moving portion 432 such that the discharge electrode supporting portion 431 is lowered by the discharge electrode moving portion 432. Therefore, the increased spaced apart distance SD, which occurs due to the lowering of liquid level caused by to the evaporation of the liquid, can be maintained within the predetermined distance range. When a large amount of the liquid 110 is accommodated or replenished into the dust collecting tank 100, the spaced apart distance SD may be reduced. In such a case, the discharge electrode supporting portion 431 can be raised by driving the discharge electrode moving portion 432, and the spaced apart distance SD can be maintained within the predetermined distance range.

The discharge electrode moving portion 432 may include a power source 433 and a connection portion 434 interconnecting the power source 433 and the discharge electrode supporting portion 431. The power source 433 may be an electric motor such as a step motor or a DC motor. Alternatively, the power source 433 may be a power generating source such as a biometal, a shape memory alloy, an actuator, or an artificial muscle. The connection portion 434 may be coupled to a rotary shaft or an actuation shaft provided in the power source 433.

By way of example, the connection portion 434 may include a rotator coupled to the rotary shaft of the power source 433 and rotated clockwise or counterclockwise by the power source 433, and a wire interconnecting the rotator and the discharge electrode supporting portion 431. The rotator may have a cylindrical shape or a ring shape. An upper end of the wire may be coupled to the rotator, and a lower end of the wire may be coupled to the discharge electrode supporting portion 431. A portion including the upper end of the wire may be wound around the rotator. As the rotator is rotated by a rotation force of the power source 433, the wire can lower or raise the discharge electrode supporting portion 431. Accordingly, the discharge electrode moving portion 432 may be configured to raise and lower the discharge electrode supporting portion 431 by the wire. As another example, the rotator may be a pulley around which the wire is wound. Further, the connection portion 434 interconnecting the power source 433 and the discharge electrode supporting portion 431 may have a raising and lowering structure that does not use a wire.

According to another embodiment, the plurality of discharge electrodes 510 may be coupled to the air discharging tube 420 so as to protrude in the radially outward direction of the central axis CA of the air discharging tube 420 while passing through the air discharging tube 420, and so as to be moved in the radially outward direction and the radially inward direction through the air discharging tube 420. In such an example, inward end portions of the discharge electrodes 510 may be connected to the above-mentioned power source 433, and the protrusion extent of the discharge electrodes 510 may be adjusted by an operation of the power source 433. As the controller 700 controls the power source 433, the protrusion extent of the discharge electrodes 510 is changed and the spaced apart distance can be maintained within the predetermined distance range thereby.

According to another embodiment, the air discharging portion 400 may be configured to adjust the spaced apart distance SD by vertically moving the air discharging tube 420. By way of example, the air discharging tube 420 may have a screw thread in an upper end portion thereof, and the cover portion 410 may have a screw thread corresponding to the screw thread of the air discharging tube 420. By rotating the air discharging tube 420 about the central axis CA, a protrusion length of the air discharging tube 420 with respect to the cover portion 410 can be adjusted. Thus, when the level of the liquid 110 in the dust collecting tank 100 is lowered and the spaced apart distance SD is changed, the height of the air discharging tube 420 and the spaced apart distance SD can be adjusted by the rotation of the air discharging tube 420. By way of example, the height adjustment of the air discharging tube 420 may be performed manually. By way of another example, a power source and a gear device for rotating the air discharging tube 420 may be disposed in the upper portion of the air discharging tube 420 and the cover portion 410, and the power source may be controlled by the controller to adjust the height of the air discharging tube.

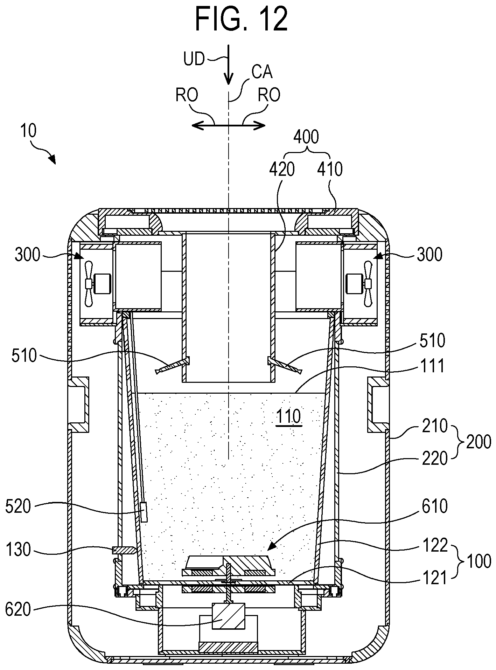

FIGS. 12 to 14 show other examples of arrangement of discharge electrodes in an air cleaner according to one embodiment. Reference is made to FIGS. 12 to 14 together in the following description.

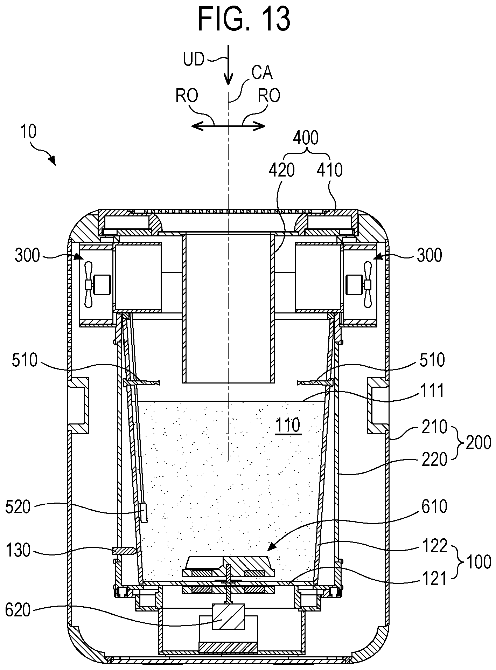

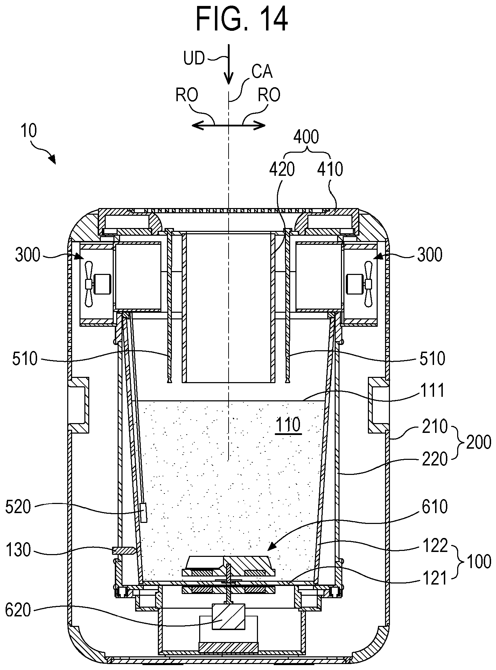

A plurality of discharge electrodes are disposed along the circumference of the air discharging tube, and are charged with a polarity opposite to the polarity with which the charging portion charges the liquid. Referring to FIG. 12, the plurality of discharge electrodes 510 are coupled to the air discharging tube 420 so as to protrude from the outer peripheral surface of the air discharging tube 420 in an oblique direction between the radially outward direction RO and a downward direction UD. Referring to FIG. 13, the plurality of discharge electrodes 510 are coupled to the dust collecting tank 100 so as to protrude from the side wall 122 of the dust collecting tank 100 toward the lower end of the air discharging tube 420. The tip portions of the discharge electrodes 510 may be positioned at the same level as the lower end of the air discharging tube 420. Referring to FIG. 14, the plurality of discharge electrodes 510 are coupled to the air discharging portion 400, specifically, the cover portion 410 or the upper end portion of the air discharging tube 420, and protrude in the downward direction UD. The discharge electrodes 510 are spaced apart from the outer peripheral surface of the air discharging tube 420 in the radially outward direction RO, and the tip portions of the discharge electrodes 510 may be positioned at the same level as the lower end of the air discharging tube 420. In the embodiments shown in FIGS. 12 to 14, the spaced apart distance between the surface 111 of the liquid and the discharge electrodes 510 can be maintained within the predetermined distance range by the above-described method. For example, as described above, the spaced apart distance can be maintained by increasing the rotation speed of the liquid, by supplying the replenishing liquid to the dust collecting tank, or by moving the discharge electrodes.

In the air cleaner according to one embodiment, the charged liquid in the dust collecting tank, and the protruding discharge electrodes disposed along the circumference of the air discharging tube and charged with a polarity different from that of the liquid form an electric field therebetween, and the charged liquid collects the pollutants ionized with a polarity different from the polarity of the liquid. Therefore, the air cleaner according to one embodiment can exhibit high air purifying performance with low power consumption in a wet environment, and can exclude a user's inconvenience such as replacement of a filter or a dust collector.

In the air cleaner according to one embodiment, the rotary vane installed on the bottom of the dust collecting tank forms the vortex in the charged liquid, thereby increasing the contact area between the surface of the charged liquid and the air. Therefore, the air cleaner according to one embodiment can prevent a decrease in dust collection performance caused by the accumulation of the pollutants, can modify the surface of the charged liquid, and can prevent a short circuit caused by the pollutants between the discharge electrodes and the liquid.

The air cleaner according to one embodiment can maintain the spaced apart distance between the surface of the charged liquid and the discharge electrodes within the distance range suitable for maintaining the ionized pollutants, and can change the revolution number of the rotary motor or change the position of the discharge electrodes with respect to the liquid in order to maintain the spaced apart distance.

The technical idea of the present disclosure has been described heretofore with reference to some embodiments and examples shown in the accompanying drawings. However, it is to be understood that various substitutions, modifications and alterations may be made without departing from the technical idea and scope of the present disclosure that can be understood by those of ordinary skill in the technical field to which the present disclosure pertains. Further, it is to be understood that such substitutions, modifications and alterations fall within the scope of the appended claims.

## Claims

1. An electrical discharge type air cleaner (10) comprising:
a dust collecting tank (100) configured to accommodate a liquid (110);
a housing (200) configured to accommodate the dust collecting tank (100);
an air supplying portion (300) configured to supply air from an outside into the dust collecting tank (100) to collect pollutants contained in the air;
an air discharging portion (400) having an air discharging tube (420) disposed so as to face a surface (111) of the liquid (110), and configured to discharge the air supplied to the dust collecting tank (100) to the outside;
a plurality of discharge electrodes (510) disposed along a circumference of the air discharging tube (420);
a charging portion (520) disposed in the dust collecting tank (100) and charging the liquid with a polarity opposite to a polarity of the plurality of discharge electrodes (510) such that an electric field is formed between the plurality of discharge electrodes (510) and the liquid (110) and the pollutants are collected into the liquid; and
a rotary vane (610) installed on a bottom of the dust collecting tank (100) and configured to rotate the liquid (110) such that a vortex is formed in the liquid,
wherein the air cleaner is configured so that a spaced apart distance between the surface (111) of the liquid (110) and the plurality of discharge electrodes (510) is within a predetermined distance range.

2. The electrical discharge type air cleaner of Claim 1, wherein the plurality of discharge electrodes (510) are configured to be charged with a positive (+) polarity, and the charging portion (520) is configured to charge the liquid with a negative (-) polarity.

3. The electrical discharge type air cleaner of Claim 1, wherein each of the plurality of discharge electrodes (510) protrudes from the air discharging tube (420) in a radially outward direction of a central axis of the air discharging tube.

4. The electrical discharge type air cleaner of Claim 1, further comprising a grounding portion (130) installed in the housing (200) and configured to ground the dust collecting tank (100).

5. The electrical discharge type air cleaner of Claim 4, wherein the dust collecting tank (100) includes a non-conductive material in an inner peripheral surface or an outer peripheral surface of the dust collecting tank.

6. The electrical discharge type air cleaner of Claim 1, further comprising a rotary motor (620) installed in the housing (200) and configured to rotate the rotary vane (610),
wherein the rotary motor (620) is configured to change a revolution number of the rotary motor so that the spaced apart distance is within the predetermined distance range.

7. The electrical discharge type air cleaner of Claim 1, further comprising:
a rotary motor (620) installed in the housing (200) and configured to rotate the rotary vane (610); and
a controller (700) configured to control the rotary motor (620),
wherein the rotary motor (620) is configured to detect a revolution number of the rotary motor and to transmit a detected revolution number to the controller (700), and
wherein the controller (700) is configured to: compare a reference revolution number of the rotary motor (620), at which the spaced apart distance is allowed to be within the predetermined distance range, with the detected revolution number inputted from the rotary motor; and to increase the revolution number of the rotary motor to heighten a level of the liquid (110) so that the spaced apart distance is within the predetermined distance range.

8. The electrical discharge type air cleaner of Claim 1, further comprising:
a liquid level detecting sensor (810) configured to detect a level of the liquid (110); and
a controller (700) configured to adjust the spaced apart distance based on a detection signal from the liquid level detecting sensor (810) so that the spaced apart distance is within the predetermined distance range.

9. The electrical discharge type air cleaner of Claim 8, further comprising a rotary motor (620) installed in the housing (200) and configured to rotate the rotary vane (610),
wherein the controller (700) is configured to change a revolution number of the rotary motor (620) so that the spaced apart distance is within the predetermined distance range.

10. The electrical discharge type air cleaner of Claim 8, further comprising:
a replenishing tank (821) configured to accommodate a replenishing liquid for replenishing the liquid (110) in the dust collecting tank (100); and
a pump (822) configured to supply the replenishing liquid of the replenishing tank to the dust collecting tank,
wherein the controller (700) is configured to control the pump (822) so that the spaced apart distance is within the predetermined distance range.

11. The electrical discharge type air cleaner of Claim 8, further comprising:
a replenishing tank (831) configured to accommodate a replenishing liquid having a concentration different from a concentration of the liquid (110);
a connection portion (832) interconnecting the dust collecting tank (100) and the replenishing tank;
a semipermeable membrane (833) installed in the connection portion; and
a valve (834) installed in the connection portion and configured to open and close the connection portion,
wherein the controller (700) is configured to control the valve (834) so that the spaced apart distance is within the predetermined distance range.

12. The electrical discharge type air cleaner of Claim 8, wherein the controller (700) is configured to change positions of the plurality of discharge electrodes (510).

13. The electrical discharge type air cleaner of Claim 12, wherein the plurality of discharge electrodes (510) are configured to be vertically movable so that the spaced apart distance is within the predetermined distance range.
